# EUROPÄISCHE PATENTSCHRIFT

(11) **EP 0 483 057 B1**
(45) Veröffentlichungstag und Bekanntmachung des Hinweises auf die Patenterteilung: **18.01.1995**
(21) Anmeldenummer: 91810768.1
(22) Anmeldetag: 30.09.1991
(51) Int. Cl.: A61F 2/30, A61F 2/36

(54) **Zentrierelement für das Schaftende einer Gelenkendoprothese**
Centraliser for the end of a joint prosthesis stem
Centralisateur pour la fin de la tige d'une endoprothèse d'articulation

(30) Priorität: 25.10.1990 CH 3398/90
(43) Veröffentlichungstag der Anmeldung: 29.04.1992
(73) Patentinhaber: SULZER Medizinaltechnik AG, 8404 Winterthur (CH); PROTEK AG, 3110 Münsingen-Bern (CH)
(72) Erfinder: Morscher, Erwin Walter, Prof. Dr.-med., Felix Platter Spital, CH-4055 Basel (CH); Willi, Roland, CH-8413 Neftenbach (CH); Koch, Rudolf, CH-8267 Berlingen (CH)
(74) Vertreter: Trieblnig, Adolf

(56) Entgegenhaltungen:
- EP-A- 0 290 735
- EP-A- 0 315 283
- EP-A- 0 427 444
- DE-A- 3 314 210
- FR-A- 2 632 182
- GB-A- 2 104 391

## Beschreibung

Die Erfindung betrifft ein Zentrierelement für das freie Schaftende einer Gelenkendoprothese, welches Element im operativ geschaffenen Knochenhohlraum fixierbar und mit dem freien Schaftende der Prothese verbindbar ist.

Um bei der Implantation einer Endoprothese um den Schaft herum ein Knochenzementbett mit in Umfangsrichtung möglichst gleichmässiger Dicke zu erhalten, sind Zentrierelemente bekannt, durch die das freie Ende des Schaftes im Operationshohlraum zentriert wird, vgl. GB-A-2 104 391.

Ein solches Zentrierelement für das distale Schaftende einer Femurkopfprothese wird - neben einem ähnlichen Element für den proximalen Bereich - in der EP-A 0 315 283 gezeigt und beschrieben. Die bekannten Zentrierelemente sind bisher derart ausgeführt worden, dass sie - um gleichzeitig als Zementsperre zu dienen - den ganzen Querschnitt des Operationshohlraumes ausfüllen; sie müssen daher vor dem Einbringen des Knochenzementes in den Knochen eingesetzt werden. Für Implantiertechniken, bei denen - wie allgemein üblich - die Prothese und das Element für ihre distale Zentrierung in den mit Knochenzement gefüllten Hohlraum eingesetzt werden, sind diese Zentrierelemente nicht geeignet.

Aufgabe der Erfindung ist es, ein Zentrierelement zu schaffen, das zusammen mit der zugehörigen Prothese implantiert wird, nachdem bereits Knochenzement für die Verankerung in den Hohlraum eingebracht worden ist. Diese Aufgabe wird mit der Erfindung dadurch gelöst, dass das Element als dreiseitige Pyramide mit einem gleichseitigen Dreieck als Basis ausgebildet ist, an der Mittel für die Verbindung mit dem Prothesenschaft vorgesehen sind.

Beim Einsetzen eines Schaftes mit darauf aufgestecktem Zentrierelement wird der Knochenzement, der zusätzlich am Ende des operativ geschaffenen Hohlraumes durch eine bekannte Zement- oder Markraumsperre am weiteren Eindringen in den Knochen gehindert wird, an den Seiten der dreiseitigen Zentrierelement-"Pyramide" entlang in den Raum zwischen Schaft und Knochen verdrängt, so dass Schaft und Zentrierelement ohne Schwierigkeiten in den zementgefüllten Hohlraum einsetzbar sind.

Der Fluss des Knochenzementes und das Einsetzen des Elementes werden erleichtert, wenn die Seiten der Pyramide konkav gekrümmt sind und/oder die Spitze der Pyramide abgerundet ist.

Als Verbindung von Prothesenschaft und Zentrierelement hat sich bewährt, wenn das Element durch einen aus seiner Basis hervorstehenden Zapfen mit der Prothese verbunden ist, der in eine entsprechende Öffnung im freien Ende des Schaftes einsteckbar ist, wobei weiterhin Zapfen und Bohrung konzentrisch mit der Pyramide in der Längsachse des Prothesenschaftes angeordnet sein können.

Bei einer Femurkopfprothese ist es wichtig, dass eine Ecke des Basisdreiecks in die laterale Richtung weist, um dort den Zementanteil in einer möglichst definierten Dicke halten zu können. Dies kann beispielsweise erreicht werden, wenn eine Ecke des Basisdreiecks einen aus der Basis hervorspringenden, nasenartigen Ansatz aufweist, der an einer nach lateral gerichteten Anlegefläche am distalen Ende des Protheseschaftes anliegt. Nase und Anlegefläche können dabei zusätzlich als Verdrehsicherung für den Schaft dienen.

Im folgenden wird die Erfindung anhand eines Ausführungsbeispiels im Zusammenhang mit der Zeichnung näher erläutert.
- Fig. 1: zeigt teilweise im Schnitt einen Schaft einer Femurkopfprothese, der, mit dem neuen Zentrierelement verbunden, in einen Knochen eingesetzt ist;
- Fig. 2: ist der Schnitt II-II von Fig. 1.

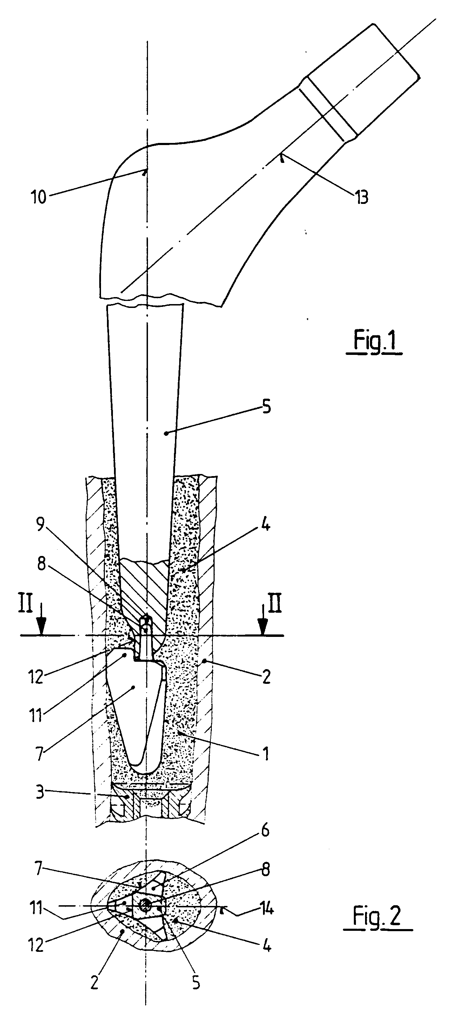

In einen operativ geschaffenen Hohlraum 1 (Fig. 1) eines Röhrenknochens 2, beispielsweise eines Femurs, ist an seinem am weitesten innen gelegenen Ende eine Markraum- oder Zementsperre 3 eingesetzt, durch die - wie erwähnt - beim Einsetzen des Prothesenschaftes 5 in den zementgefüllten Hohlraum 1 ein Ausweichen von Knochenzement 4 in den Knochen 2 hinein verhindert wird.

In den Knochenzement 4 ist der Schaft 5 eingesetzt, der an seinem - im Falle einer Femurkopfprothese - distalen Ende ein Zentrierelement 6 trägt. Das Element 6 hat im wesentlichen die Form einer dreiseitigen Pyramide, deren Basis ein gleichseitiges Dreieck (Fig. 2) und deren abgerundete Spitze zur Zementsperre 3 hin gerichtet ist. Weiterhin sind, wie Fig. 2 erkennen lässt, die Seiten 7 der Pyramide konkav gekrümmt, wodurch der "freie Querschnitt" für den beschriebenen "Fluss" des Zementes vergrössert wird.

Die Verbindung des Elementes 6 mit dem Schaft 5 besteht in einem, aus dem Zentrum des Basisdreiecks herausragenden Zapfen 8, der in eine entsprechende Bohrung 9 im distalen Ende des Schaftes 5 eingesteckt ist. Der Zapfen 8, der zur Verbesserung der "Haftung" der Steckverbindung leicht konisch ausgeführt ist, und die Bohrung 9 sind dabei konzentrisch mit der Längsachse 10 des Schafts 5 angeordnet, was die Zentrierung des Schaftes erleichtert. Selbstverständlich sind auch eine Umkehrung der Anordnung von Zapfen und Bohrung oder andere Konstruktionen für eine Verbindung von Schaft 5 und Element 6 möglich.

Wie bereits erwähnt, ist es bei einer Femurkopfprothese wichtig, dass eine Ecke der dreiseitigen Pyramide des Zentrierelementes 6 in Richtung lateral weist. Um dies zu gewährleisten, überragt an einer Ecke ein nasenartiger Ansatz 11 das Basisdreieck des Elementes 6; dieser Ansatz 11 stützt sich an einer Anlagefläche 12 des Schaftes 5 ab, die an seinem distalen Ende vorgesehen ist. Dass die Flächennormale dieser Auflagefläche 12 nach lateral gerichtet ist, zeigt ihre Lage relativ zu der in Fig. 2 angedeuteten, durch die Längsachse 10 und die Prothesenhalsachse 13 (Fig. 1) definierten Mittelebene 14, die mit der Darstellungsebene der Fig. 1 übereinstimmt. Auch hier können andere bekannte konstruktive Mittel für die Ausrichtung einer Ecke des Basisdreieckes der Pyramide nach lateral angewendet werden.

## Patentansprüche

1. Zentrierelement für das freie Schaftende einer Gelenkendoprothese, welches Element (6) im operativ geschaffenen Knochenhohlraum (1) fixierbar und mit dem freien Schaftende der Prothese verbindbar ist, dadurch gekennzeichnet, dass das Element (6) als dreiseitige Pyramide mit einem gleichseitigen Dreieck als Basis ausgebildet ist, an der Mittel (8, 9) für die Verbindung mit dem Prothesenschaft (5) vorgesehen sind.

2. Zentrierelement nach Anspruch 1, dadurch gekennzeichnet, dass die Seiten (7) der Pyramide konkav gekrümmt sind.

3. Zentrierelement nach Anspruch 1 oder 2, dadurch gekennzeichnet, dass die Spitze der Pyramide abgerundet ist.

4. Zentrierelement nach einem der Ansprüche 1 bis 3, dadurch gekennzeichnet, dass das Element (6) durch einen aus seiner Basis hervorstehenden Zapfen (8) mit der Prothese verbunden ist, der in eine entsprechende Öffnung (9) im freien Ende des Schaftes (5) einsteckbar ist.

5. Zentrierelement nach Anspruch 4, dadurch gekennzeichnet, dass Zapfen (8) und Bohrung (9) konzentrisch mit der Pyramide in der Längsachse (10) des Prothesenschaftes (5) angeordnet sind.

6. Zentrierelement nach einem der Ansprüche 1 bis 5 für eine Femurkopfprothese, dadurch gekennzeichnet, dass Mittel (11,12) vorgesehen sind, die eine Ausrichtung einer Ecke des Dreieckes in die laterale Richtung gewährleisten.

7. Zentrierelement nach Anspruch 6, dadurch gekennzeichnet, dass eine Ecke des Basisdreiecks einen aus der Basis hervorspringenden, nasenartigen Ansatz (11) aufweist, der an einer nach lateral gerichteten Anlegefläche (12) am distalen Ende des Protheseschaftes (5) anliegt.

## Claims

1. A centreing element for the free end of the shank of a joint endoprosthesis, where the said element (6) may be fixed in a cavity (1) created operatively in the bone and be connected to the free end of the shank of the proshesis, characterized in that the element (6) is made as a three-sided pyramid with an equilateral triangular base, on which means (8, 9) are provided for connection to the shank (5) of the prosthesis.

2. A centreing element as in Claim 1, characterized in that the sides of the pyramid have a concave curvature.

3. A centreing element as in Claim 1 or 2, characterized in that the apex of the pyramid is rounded.

4. A centreing element as in one of the Claims 1 to 3, characterized in that the element (6) is connected to the prosthesis through a stud (8) which projects from its base and may be plugged into a corresponding opening (9) in the free end of the shank (5).

5. A centreing element as in Claim 1, characterized in that the stud (8) and the hole (9) are arranged to be concentric with the pyramid on the longitudinal axis (10) of the shank (5) of the prosthesis.

6. A centreing element as in one of the Claims 1 to 5 for a femur head prosthesis, characterized in that means (11, 12) are provided which guarantee alignment of one corner of the triangle in the lateral direction.

7. A centreing element as in Claim 6, characterized in that one corner of the base triangle exhibits a noselike extension (11) which projects from the base and rests against a laterally-directed contact area (12) at the distal end of the shank (5) of the prosthesis.

## Revendications

1. Elément de centrage pour l'extrémité libre de la tige d'une endoprothèse articulaire, ledit élément (6) pouvant être immobilisé dans la cavité osseuse (1) créée par voie opératoire et pouvant être relié à l'extrémité libre de la tige de la prothèse, caractérisé en ce que l'élément (6) est conçu sous la forme d'une pyramide triangulaire dont la base est formée par un triangle équilatéral et sur laquelle sont prévus des moyens (8, 9) pour réaliser la liaison avec la tige (5) de la prothèse.

2. Elément de centrage selon la revendication 1, caractérisé en ce que les côtés (7) de la pyramide possèdent une courbure concave.

3. Elément de centrage selon la revendication 1 ou 2, caractérisé en ce que le sommet de la pyramide est arrondi.

4. Elément de centrage selon l'une des revendications 1 à 3, caractérisé en ce que l'élément (6) est relié à la prothèse par un téton (8) qui fait saillie sur sa base et qui peut être emmanché dans une ouverture correspondante (9) ménagée à l'extrémité libre de la tige (5).

5. Elément de centrage selon la revendication 4, caractérisé en ce que le téton (8) et l'alésage (9) sont disposés concentriquement à la pyramide dans l'axe longitudinal (10) de la tige (5) de la prothèse.

6. Elément de centrage selon l'une des revendications 1 à 5, pour une prothèse de tête fémorale, caractérisé en ce qu'il est prévu des moyens (11, 12) qui garantissent une orientation d'un angle du triangle dans la direction latérale.

7. Elément de centrage selon la revendication 6, caractérisé en ce qu'un angle du triangle de base comporte une surépaisseur en forme de talon (11) qui fait saillie sur la base et qui est appliquée contre une surface d'appui (12) orientée latéralement et située à l'extrémité distale de la tige (5) de la prothèse.
